# EUROPEAN PATENT APPLICATION

(11) **EP 2 343 053 A1**
(43) Date of publication of application: **13.07.2011**
(21) Application number: 11163168.5
(22) Date of filing: 22.05.2007
(51) Int. Cl.: A61K 9/14

(54) **Nanoparticulate posaconazole formulations**

(30) Priority: 30.05.2006 US 808961 P
(62) Divisional of application: 07784042.9
(71) Applicant: Elan Pharma International Limited, County Westmeath (IE)
(72) Inventor: The designation of the inventor has not yet been filed
(74) Representative: Wichmann, Hendrik

(57) **Abstract**

The invention is directed to compositions comprising a nanoparticulate posaconazole, or a salt or derivative thereof, having improved bioavailability. The nanoparticulate posaconazole particles of the composition have an effective average particle size of less than about 2000 nm and are useful in the prevention and treatment of fungal infection and related diseases. The posaconazole particles may be formulated as a parenteral dosage form.

## Description

This is a divisional application on the basis of the parent application European Patent Application 07 784 042.9 the content of which is incorporated herein by reference.

This application claims the benefit under 35 U.S.C. § 119(e) to U.S. Provisional Application No. 60/808,961, filed on May 30, 2006, which is incorporated by reference herein in its entirety.

### FIELD OF INVENTION

The present invention relates generally to compounds and compositions useful in the prevention and treatment of fungal infections and related diseases. More specifically, the invention relates to compositions comprising nanoparticulate posaconazole, or a salt or derivative thereof. The nanoparticulate posaconazole compositions comprise posaconazole particles having an effective average particle size of less than about 2000 nm. The compositions of the invention may also comprise any number of polymeric materials for a controlled and/or delayed release formulation.

### BACKGROUND OF INVENTION

### Background Regarding Posaconazole

Posaconazole, CAS No. 171228-49-2, is a triazole that is structurally related to itraconazole. It is being developed by Schering-Plough Pharmaceuticals, formerly known as SCH 56592, and is currently in Phase III trials.

Posaconazole has the following chemical structure:

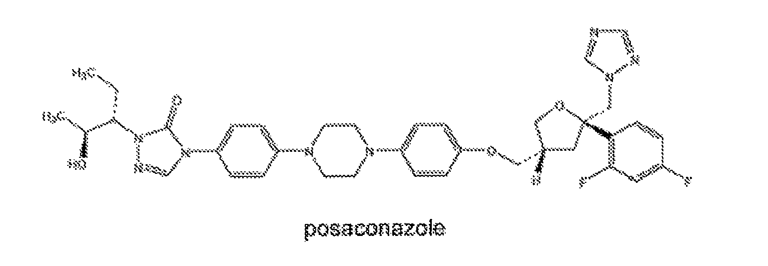

Posaconazole is a potent broad-spectrum azole antifungal agent useful in the treatment of invasive fungal infections. Like other azole antifungal agents, posaconazole works principally by inhibition of cytochrome P450 14a-demethylase (P45014DM). This enzyme is in the sterol biosynthesis pathway that leads from lanosterol to ergosterol. Compared to itraconazole, posaconazole is a significantly more potent inhibitor of sterol C 14 demethylation, particularly in *Aspergillus.* Posaconazole has a broad spectrum of activity against opportunistic fungal infections. Representative fungal agents that posaconazole is generally potent against include *Candida spp., Cryptococcus neoformans, Aspergillus spp., Rhizopus spp., Blastomyces dermatitidis, Coccidioides immitis, Histoplasma capsulatum, dermatophytes* and *dematiaceous* fungi.

Posaconazole has been formulated in oral tablet and suspension preparations. Posaconazole is undergoing Phase III clinical trials, and if successful, should be marketed by Schering-Plough of Kenilworth, New Jersey under the trade name Noxafil®. Representative dosing of posaconazole includes 440 mg twice daily for 3 days, followed by 400 mg daily or twice daily for 25 days. Posaconazole is a lipophilic drug with high permeability (>10-5 cm/s), low aqueous solubility (<1 µg/ml), and a pKa of 3.6 for the piperazine nitrogen and 4.6 for the triazole nitrogen. Consumption of a meal containing fat calories increases the relative oral bioavailability of posaconazole by approximately 400% regardless of which formulation is administered (tablet versus suspension).

Posaconazole compounds have been described in U.S. Pat. Appl. No. 2003/0055067 for "Antifungal Composition with Enhanced Bioavailability," U.S. Pat. Appl. No. 2004/0058974 for "Treating Fungal Infections," and European Patent Publication 1372394 (A1) for "Liquid Suspensions of Posaconazole (SCH 56592) with Enhanced Bioavailability for Treating Fungal Infections." These patent publications are hereby incorporated by reference.

### Background Regarding Nanoparticulate Active Agent Compositions

Nanoparticulate active agent compositions, first described in U.S. Patent No. 5,145,684 ("the '684 patent"), are particles consisting of a poorly soluble therapeutic or diagnostic agent having adsorbed onto or associated with the surface thereof a non-crosslinked surface stabilizer. The '684 patent does not describe nanoparticulate compositions of posaconazole.

Methods of making nanoparticulate active agent compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles." Nanoparticulate active agent compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill;" 6,432,381 for "Methods for Targeting Drug Delivery to the Upper and/or Lower Gastrointestinal Tract," 6,592,903 for "Nanoparticulate Dispersions Comprising a Synergistic Combination of a Polymeric Surface Stabilizer and Dioctyl Sodium Sulfosuccinate," 6,582,285 for "Apparatus for sanitary wet milling;" 6,656,504 for "Nanoparticulate Compositions Comprising Amorphous Cyclosporine;" 6,742,734 for "System and Method for Milling Materials;" 6,745,962 for "Small Scale Mill and Method Thereof;" 6,811,767 for "Liquid droplet aerosols of nanoparticulate drugs;" 6,908,626 for "Compositions having a combination of immediate release and controlled release characteristics;" 6,969,529 for "Nanoparticulate compositions comprising copolymers of vinyl pyrrolidone and vinyl acetate as surface stabilizers;" 6,976,647 for "System and Method for Milling Materials," 6,991,191 for "Method of using a small scale mill;" 7,101,576 for "Nanoparticulate megestrol formulations;" and 7,198,795 for "In vitro methods for evaluating the in vivo effectiveness of dosage forms of microparticulate of nanoparticulate active agent compositions," all of which are specifically incorporated by reference.

In addition, U.S. Patent Publication No. 20070104792 A1 for "Nanoparticulate tadalafil formulations;" U.S. Patent Publication No. 20070098805 A1 for "Methods of making and using novel griseofulvin compositions;" U.S. Patent Publication No. 20070065374 A1 for "Nanoparticulate leukotriene receptor antagonist/corticosteroid formulations;" U.S. Patent Publication No. 20070059371 A1 for "Nanoparticulate ebastine formulations;" U.S. Patent Publication No. 20070048378 A1 for "Nanoparticulate anticonvulsant and immunosuppressive compositions;" U.S. Patent Publication No. 20070042049 A1 for "Nanoparticulate benidipine compositions;" U.S. Patent Publication No. 20070015719 A1 for "Nanoparticulate clarithromycin formulations;" U.S. Patent Publication No. 20070003628 for "Nanoparticulate clopidogrel formulations;" U.S. Patent Publication No. 20070003615 for "Nanoparticulate clopidogrel and aspirin combination formulations;" U.S. Patent Publication No. 20060292214 A1 for "Nanoparticulate acetaminophen formulations;" U.S. Patent Publication No. 20060275372 A1 for "Nanoparticulate imatinib mesylate formulations;" U.S. Patent Publication No. 20060270657 A1 for "N-substituted heterocyclic sulfonamides;" U.S. Patent Publication No. 20060246142 A1 for "Nanoparticulate quinazoline derivative formulations;" U.S. Patent Publication No. 20060246141 A1 for "Nanoparticulate lipase inhibitor formulations;" U.S. Patent Publication No. 20060216353 A1 for "Nanoparticulate corticosteroid and antihistamine formulations;" U.S. Patent Publication No. 20060210639 A1 for "Nanoparticulate bisphosphonate compositions;" U.S. Patent Publication No. 20060210638 A1 for "Injectable compositions of nanoparticulate immunosuppressive compounds;" U.S. Patent Publication No. 20060204588 A1 for "Formulations of a nanoparticulate finasteride, dutasteride or tamsulosin hydrochloride, and mixtures thereof;" U.S. Patent Publication No. 20060198896 A1 for "Aerosol and injectable formulations of nanoparticulate benzodiazepine;" U.S. Patent Publication No. 20060193920 A1 for "Nanoparticulate Compositions of Mitogen-Activated Protein (MAP) Kinase Inhibitors;" U.S. Patent Publication No. 20060188566 for "Nanoparticulate formulations of docetaxel and analogues thereof;" U.S. Patent Publication No. 20060165806 A1 for "Nanoparticulate candesartan formulations;" U.S. Patent Publication No. 20060159767 A1 for "Nanoparticulate bicalutamide formulations;" U.S. Patent Publication No. 20060159766 A1 for "Nanoparticulate tacrolimus formulations;" U.S. Patent Publication No. 20060159628 A1 for "Nanoparticulate benzothiophene formulations;" U.S. Patent Publication No. 20060154918 A1 for "Injectable nanoparticulate olanzapine formulations;" U.S. Patent Publication No. 20060121112 A1 for "Topiramate pharmaceutical composition;" U.S. Patent Publication No. 20020012675 A1, for "Controlled Release Nanoparticulate Compositions;" U.S. Patent Publication No. 20050276974 for "Nanoparticulate Fibrate Formulations;" U.S. Patent Publication No. 20050238725 for "Nanoparticulate compositions having a peptide as a surface stabilizer;" U.S. Patent Publication No. 20050233001 for "Nanoparticulate megestrol formulations;" U.S. Patent Publication No. 20050147664 for "Compositions comprising antibodies and methods of using the same for targeting nanoparticulate active agent delivery;" U.S. Patent Publication No. 20050063913 for "Novel metaxalone compositions;" U.S. Patent Publication No. 20050042177 for "Novel compositions of sildenafil free base;" U.S. Patent Publication No. 20050031691 for "Gel stabilized nanoparticulate active agent compositions;" U.S. Patent Publication No. 20050019412 for " Novel glipizide compositions;" U.S. Patent Publication No. 20050004049 for "Novel griseofulvin compositions;" U.S. Patent Publication No. 20040258758 for "Nanoparticulate topiramate formulations;" U.S. Patent Publication No. 20040258757 for " Liquid dosage compositions of stable nanoparticulate active agents;" U.S. Patent Publication No. 20040229038 for "Nanoparticulate meloxicam formulations;" U.S. Patent Publication No. 20040208833 for "Novel fluticasone formulations;" U.S. Patent Publication No. 20040195413 for " Compositions and method for milling materials;" U.S. Patent Publication No. 20040156895 for "Solid dosage forms comprising pullulan;" U.S. Patent Publication No. U.S. Patent Publication No. U.S. Patent Publication No. 20040156872 for "Novel nimesulide compositions;" U.S. Patent Publication No. 20040141925 for "Novel triamcinolone compositions;" U.S. Patent Publication No. 20040115134 for novel nifedipine compositions;" U.S. Patent Publication No. 20040105889 for "Low viscosity liquid dosage forms;" U.S. Patent Publication No. 20040105778 for "Gamma irradiation of solid nanoparticulate active agents;" U.S. Patent Publication No. 20040101566 for "Novel benzoyl peroxide compositions;" U.S. Patent Publication No. 20040057905 for "Nanoparticulate beclomethasone dipropionate compositions;" U.S. Patent Publication No. 20040033267 for "Nanoparticulate compositions of angiogenesis inhibitors;" U.S. Patent Publication No. 20040033202 for "Nanoparticulate sterol formulations and novel sterol combinations;" U.S. Patent Publication No. 20040018242 for "Nanoparticulate nystatin formulations;" U.S. Patent Publication No. 20040015134 for "Drug delivery systems and methods;" U.S. Patent Publication No. 20030232796 for "Nanoparticulate polycosanol formulations & novel polycosanol combinations;" U.S. Patent Publication No. 20030215502 for "Fast dissolving dosage forms having reduced friability;" U.S. Patent Publication No. 20030185869 for "Nanoparticulate compositions having lysozyme as a surface stabilizer;" U.S. Patent Publication No. 20030181411 for "Nanoparticulate compositions of mitogen-activated protein (MAP) kinase inhibitors;" U.S. Patent Publication No. 20030137067 for "Compositions having a combination of immediate release and controlled release characteristics;" U.S. Patent Publication No. 20030108616 for "Nanoparticulate compositions comprising copolymers of vinyl pyrrolidone and vinyl acetate as surface stabilizers;" U.S. Patent Publication No. 20030095928 for "Nanoparticulate insulin;" U.S. Patent Publication No. 20030087308 for "Method for high through put screening using a small scale mill or microfluidics;" U.S. Patent Publication No. 20030023203 for "Drug delivery systems & methods;" U.S. Patent Publication No. 20020179758 for "System and method for milling materials; and U.S. Patent Publication No. 20010053664 for "Apparatus for sanitary wet milling," describe nanoparticulate active agent compositions and are specifically incorporated by reference.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." These are also incorporated herein by reference.

Posaconazole has high therapeutic value in the prevention and treatment of fungal infections and related diseases. However, because posaconazole dosing is highly susceptible to food intake, significant bioavailability can be problematic. There is a need in the art for posaconazole formulations which overcome this and other problems associated with the use of posaconazole in the prevention and treatment of fungal infections and related diseases. The present invention satisfies this need by providing an improved dissolution rate of posaconazole. The improved dissolution rate results in the enhanced bioavailability in the fasting state that would match that seen in the fed state and eliminate the requirement to take the posaconazole with food.

### DESCRIPTION OF THE INVENTION

The present invention relates to compositions comprising a nanoparticulate posaconazole, or a salt or derivative thereof, for the treatment of fungal infection and related diseases. Additionally, the invention provides a formulation of posaconazole suitable for parenteral administration. Conventional parenteral formulation approaches for insoluble drugs typically involve the use of potentially toxic excipients such as Cremophor or require a carrier vehicle with a highly acidic or basic pH. The parenteral posaconazole formulations of the invention eliminate these problems and allow for the manufacture of a commercially acceptable dosage form.

Moreover, the present invention further provides compositions comprising nanoparticulate posaconazole particles in combination with one or more polymeric coatings for a sustained and/or delayed controlled drug release.

The present invention relates to compositions comprising nanoparticulate posaconazole or a salt or derivative thereof. The compositions can also comprse at least one surface stabilizer adsorbed on or associated with the surface of the posaconazole particles. The nanoparticulate posaconazole particles have an effective average particle size of less than about 2,000 nm.

The present invention also relates to a controlled release formulation in which the nanoparticulate posaconazole particles are coated with one or more polymeric coatings or incorporated in a polymeric material matrix so that the active is released at a sustained and/or delayed rate of release for an improved, more consistent dissolution rate within the stomach and small intestines thereby avoiding the occurrence of localized "hot spots" of high drug concentrations.

The nanoparticulate posaconazole compositions can also be formulated as an parenteral formulation for non-oral administration immediately prior to or during an invasive fungal infection for the immediate onset of drug therapeutic action as well as improved ease of administration. Parenteral compositions may be formulated for injection or infusion such as intraarterial, intramuscular, subcutaneous, or intradermal routes of administration.

A preferred dosage form of the invention is a solid dosage form, although any pharmaceutically acceptable dosage form can be utilized.

In yet another embodiment, the invention encompasses a nanoparticulate posaconazole composition, wherein administration of the composition to a subject in a fasted state is bioequivalent to administration of the composition to a subject in a fed state.

Another aspect of the invention is directed to pharmaceutical compositions comprising a nanoparticulate posaconazole, or a salt or derivative thereof, at least one surface stabilizer, and a pharmaceutically acceptable carrier, as well as any desired excipients.

Another embodiment of the invention is directed to nanoparticulate posaconazole compositions comprising one or more additional compounds useful in the prevention and treatment of a pathological state induced by fungal infection and related diseases.

This invention further discloses a method of making the inventive nanoparticulate posaconazole compositions. Such a method comprises contacting particles of posaconazole, or a salt or derivative thereof, with at least one surface stabilizer for a time and under conditions sufficient to provide a stabilized nanoparticulate posaconazole composition, wherein the posaconazole particles have an effective average particle size of less than about 2000 nm.

The present invention is also directed to methods of treatment including but not limited to, the prevention and treatment of pathological states induced by fungal infection and related diseases, using the novel nanoparticulate posaconazole compositions disclosed herein. Such methods comprise administering to a subject a therapeutically effective amount of a nanoparticulate posaconazole composition. Other methods of treatment using the nanoparticulate compositions of the invention are known to those of skill in the art.

### DETAILED DESCRIPTION OF THE INVENTION

### I. Nanoparticulate Posaconazole Compositions

The present invention is directed to nanoparticulate compositions comprising a posaconazole, or a salt or derivative thereof. The compositions comprise particles of posaconazole, or a salt or derivative thereof, and preferably at least one surface stabilizer adsorbed on or associated with the surface of the drug. The particles of posaconazole or a salt or derivative thereof have an effective average particle size of less than about 2000 nm.

Advantages of the nanoparticulate posaconazole compositions of the invention as compared to non-nanoparticulate (e.g., microcrystalline or solubilized) posaconazole compositions include, but are not limited to: (1) smaller tablet or other solid dosage form size; (2) smaller doses of posaconazole required to obtain the same pharmacological effect; (3) enhanced bioavailability in the fasting state that would match that seen in the fed state and eliminate the requirement to take posaconazole with food; (4) improved pharmacokinetic profiles; (5) an increased rate of dissolution; (6) the posaconazole compositions can be used in conjunction with other active agents useful in the prevention and treatment of fungal infections and related diseases; and (7) the nanoparticulate posaconazol compositions can be utilized in a parenteral formulation that eliminates the need for toxic excipients or pH extremes of pH for drug solubility.

The present invention also includes nanoparticulate posaconazole, or a salt or derivative thereof, compositions together with one or more non-toxic physiologically acceptable carriers, adjuvants, or vehicles, collectively referred to as carriers. The compositions can be formulated for parenteral injection (*e.g*., intravenous, intramuscular, or subcutaneous), oral administration in solid, liquid, or aerosol form, vaginal, nasal, rectal, ocular, local (powders, ointments, or drops), buccal, intracisternal, intraperitoneal, or topical administrations, and the like.

A preferred dosage form of the invention is a solid dosage form or parenteral formulation, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred.

The present invention is described herein using several definitions, as set forth below and throughout the application.

The term "effective average particle size," as used herein, means that at least about 50% of the nanoparticulate posaconazole particles have a size of less than about 2000 nm, by weight or by other suitable measurement technique (e.g., such as by volume, number, etc.), when measured by, for example, sedimentation flow fractionation, photon correlation spectroscopy, light scattering, disk centrifugation, and other techniques known to those of skill in the art.

As used herein, "about" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term.

As used herein with reference to stable posaconazole particles, "stable" means that the particles do not appreciably flocculate or agglomerate due to interparticle attractive forces or otherwise increase in particle size. "Stable" connotes, but is not limited to one or more of the following parameters: (1) the particles do not appreciably flocculate or agglomerate due to interparticle attractive forces or otherwise significantly increase in particle size over time; (2) the physical structure of the posaconazole particles is not altered over time, such as by conversion from an amorphous phase to a crystalline phase; (3) the posaconazole particles are chemically stable; and/or (4) where the posaconazole or a salt or derivative thereof has not been subject to a heating step at or above the melting point of the posaconazole particles in the preparation of the nanoparticles of the invention.

The term "conventional" or "non-nanoparticulate active agent" shall mean an active agent which is solubilized or which has an effective average particle size of greater than about 2000 nm. Nanoparticulate active agents as defined herein have an effective average particle size of less than about 2000 nm.

The phrase "poorly water soluble drugs" as used herein refers to drugs having a solubility in water of less than about 30 mg/ml, less than about 20 mg/ml, less than about 10 mg/ml, or less than about 1 mg/ml.

As used herein, the phrase "therapeutically effective amount" shall mean that drug dosage that provides the specific pharmacological response for which the drug is administered in a significant number of subjects in need of such treatment. It is emphasized that a therapeutically effective amount of a drug that is administered to a particular subject in a particular instance will not always be effective in treating the conditions/diseases described herein, even though such dosage is deemed to be a therapeutically effective amount by those of skill in the art.

### A. Preferred Characteristics of the Nanoparticulate

### Posaconazole Compositions of the Invention

### 1. Increased Bioavailability

The nanoparticulate posaconazole, or a salt or derivative thereof, formulations of the invention are proposed to exhibit increased bioavailability, and require smaller doses as compared to prior conventional posaconazole formulations.

### 2. Improved Pharmacokinetic Profiles

The nanoparticulate posaconazole, or a salt or derivative thereof, formulations of the invention are proposed to exhibit improved pharmacokinetic profiles in which the maximum plasma concentration of posaconazole are higher for a given dose than those occurring following administration of a conventional dosage form. In addition, the time to reach maximum plasma concentration will be shorter with nanoparticulate posaconazole. These changes will improve the therapeutic efficacy of posaconazole.

The invention also provides nanoparticulate posaconazole, or a salt or derivative thereof, compositions having a desirable pharmacokinetic profile when administered to mammalian subjects. The desirable pharmacokinetic profile of the compositions comprising posaconazole includes but is not limited to: (1) a Cₘₐₓ for a posaconazole, when assayed in the plasma of a mammalian subject following administration, that is preferably greater than the Cₘₐₓ for a non-nanoparticulate formulation of the same posaconazole, administered at the same dosage; and/or (2) an AUC for posaconazole, when assayed in the plasma of a mammalian subject following administration, that is preferably greater than the AUC for a non-nanoparticulate formulation of the same posaconazole, administered at the same dosage; and/or (3) a Tₘₐₓ for posaconazole, when assayed in the plasma of a mammalian subject following administration, that is preferably less than the Tₘₐₓ for a non-nanoparticulate formulation of the same posaconazole, administered at the same dosage. The desirable pharmacokinetic profile, as used herein, is the pharmacokinetic profile measured after the initial dose of posaconazole or a salt or derivative thereof.

In one embodiment, a composition comprising a nanoparticulate posaconazole exhibits in comparative pharmacokinetic testing with a non-nanoparticulate formulation of the same posaconazole, administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate posaconazole formulation.

In another embodiment, the composition comprising a nanoparticulate posaconazole exhibits in comparative pharmacokinetic testing with a non-nanoparticulate formulation of the same posaconazole, administered at the same dosage, a Cₘₐₓ which is at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate posaconazole formulation.

In yet another embodiment, the composition comprising a nanoparticulate posaconazole exhibits in comparative pharmacokinetic testing with a non-nanoparticulate formulation of the same posaconazole, administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate posaconazole formulation.

In one embodiment of the invention, the Tₘₐₓ of posaconazole, when assayed in the plasma of the mammalian subject, is less than about 6 to about 8 hours. In other embodiments of the invention, the Tₘₐₓ of posaconazole is less than about 6 hours, less than about 5 hours, less than about 4 hours, less than about 3 hours, less than about 2 hours, less than about 1 hour, or less than about 30 minutes after administration.

The desirable pharmacokinetic profile, as used herein, is the pharmacokinetic profile measured after the initial dose of posaconazole or a salt or derivative thereof. The compositions can be formulated in any way as described herein and as known to those of skill in the art.

### 3. The Pharmacokinetic Profiles of the Posaconazole Compositions of the Invention are not Affected by the Fed or Fasted State of the Subject Ingesting the Compositions

The invention encompasses posaconazole compositions wherein the pharmacokinetic profile of the posaconazole is not substantially affected by the fed or fasted state of a subject ingesting the composition. This means that there is no substantial difference in the quantity of drug absorbed or the rate of drug absorption when the nanoparticulate posaconazole compositions are administered in the fed versus the fasted state.

Benefits of a dosage form which substantially eliminates the effect of food include an increase in subject convenience, thereby increasing subject compliance, as the subject does not need to ensure that they are taking a dose either with or without food. This is significant, as with poor subject compliance an increase in the medical condition for which the drug is being prescribed may be observed.

### 4. Bioequivalency of Posaconazole Compositions of the Invention When Administered in the Fed Versus the Fasted State

The invention also encompasses provides a nanoparticulate posaconazole composition in which administration of the composition to a subject in a fasted state is bioequivalent to administration of the composition to a subject in a fed state.

The difference in absorption (AUC) or Cₘₐₓ of the nanoparticulate posaconazole compositions of the invention, when administered in the fed versus the fasted state, preferably is less than about 60%, less than about 55%, less than about 50%, less than about 45%, less than about 40%, less than about 35%, less than about 30%, less than about 25%, less than about 20%, less than about 15%, less than about 10%, less than about 5%, or less than about 3%.

In one embodiment of the invention, the invention encompasses compositions comprising a nanoparticulate posaconazole, wherein administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% CI for AUC must be between 0.80 to 1.25 and the 90% CI for Cₘₐₓ must between 0.70 to 1.43.

### 5. Dissolution Profiles of the Posaconazole Compositions of the Invention

The nanoparticulate posaconazole, or a salt or derivative thereof, compositions of the invention are proposed to have unexpectedly dramatic dissolution profiles. Rapid dissolution of an administered active agent is preferable, as faster dissolution generally leads to faster onset of action and greater bioavailability. To improve the dissolution profile and bioavailability of the posaconazole it would be useful to increase the drug's dissolution so that it could attain a level close to 100%.

The posaconazole compositions of the invention preferably have a dissolution profile in which within about 5 minutes at least about 20% of the composition is dissolved. In other embodiments of the invention, at least about 30% or at least about 40% of the posaconazole composition is dissolved within about 5 minutes. In yet other embodiments of the invention, preferably at least 40%, at least about 50%, at least about 60%, at least about 70%, or at least about 80% of the posaconazole composition is dissolved within about 10 minutes. Finally, in another embodiment of the invention, preferably at least about 70%, at least about 80%, at least about 90%, or at least about 100% of the posaconazole composition is dissolved within 20 minutes.

Dissolution is preferably measured in a medium which is discriminating. Such a dissolution medium will produce two very different dissolution curves for two products having very different dissolution profiles in gastric juices; i.e., the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### 6. Redispersability of the Posaconazole Compositions of the Invention

An additional feature of the posaconazole, or a salt or derivative thereof, compositions of the invention is that the compositions re-disperse such that the effective average particle size of the re-dispersed posaconazole particles is less than about 2 microns. This is significant, as if upon administration the posaconazole compositions of the invention did not re-disperse to a substantially nanoparticulate size, then the dosage form may lose the benefits afforded by formulating the posaconazole into a nanoparticulate size.

This is because nanoparticulate active agent compositions benefit from the small particle size of the active agent; if the active agent does not disperse into the small particle sizes upon administration, them "clumps" or agglomerated active agent particles are formed, owing to the extremely high surface free energy of the nanoparticulate system and the thermodynamic driving force to achieve an overall reduction in free energy. With the formulation of such agglomerated particles, the bioavailability of the dosage form my fall.

In other embodiments of the invention, the redispersed posaconazole, or a salt or derivative thereof, particles of the invention have an effective average particle size of less than about less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

Moreover, the nanoparticulate posaconazole or a salt or derivative thereof compositions of the invention exhibit dramatic redispersion of the nanoparticulate posaconazole particles upon administration to a mammal, such as a human or animal, as demonstrated by reconstitution/redispersion in a biorelevant aqueous media such that the effective average particle size of the redispersed posaconazole particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. See e.g., Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (i.e., weak acids and corresponding salts of that acid), monoprotic and polyprotic electrolytes, etc.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed posaconazole or a salt or derivative thereof particles of the invention (redispersed in an aqueous, biorelevant, or any other suitable media) have an effective average particle size of less than about less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 650 nm, less than about 600 nm, less than about 550 nm, less than about 500 nm, less than about 450 nm, less than about 400 nm, less than about 350 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods. Such methods suitable for measuring effective average particle size are known to a person of ordinary skill in the art.

Redispersibility can be tested using any suitable means known in the art. See e.g., the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 7. Posaconazole Compositions Used in Conjunction with Other Active Agents

The posaconazole, or a salt or derivative thereof, compositions of the invention can additionally comprise one or more compounds useful in the prevention and treatment of fungal infection and related diseases, or the posaconazole compositions can be administered in conjunction with such a compound. Exemplary active agents that can be co-formulated or co-administered with the posaconazole formulations of the invention include, but are not limited to, steroids, antibiotics, and antifungal agents. Exemplary antifungal agents include, but are not limited to, clotrimazole (Gyne-Lotrimin®, Mycelex-7®, Lotrisone® (clotrimazole/betamethasone diproprionate)), fluconazole, ketoconazole (Nizoral®), nystatin, flucanozole (Difulcan®), itraconazole (Sporanox®), amphotericin B, butoconazole nitrate (Femstat®), griseofulvin (Gris-PEG®, Grisactin®, Fulvicin P/G®), ciclopiroz olamine (Loprox®), miconazole nitrate (Monistat®, Mycolog-II®), oxiconazole nitrate (Oxistat®), and econazole nitrate (Spectazole®).

### 8. Parenteral Nanoparticulate Posaconazole Compositions Eliminate the Need for Toxic Excipients or Carrier Vehicles With High or Low pH Extremes for Drug Solubility

Parenteral compositions may be formulated for injection or infusion such as intraarterial, intramuscular, subcutaneous, or intradermal routes of administration. Formulated in this manner, the nanoparticulate posaconazole compositions of the invention eliminate the need for toxic excipients or carrier vehicles with high or low pH extremes for solubilization of the active drug. Liquid parenteral formulations suitable for use in this manner are pharmaceutically acceptable solutions well known to those skilled in the art and aqueous-based carriers are particularly preferred.

### B. Nanoparticulate Posaconazole Compositions

The invention provides compositions comprising posaconazole, or a salt or derivative thereof, particles and at least one surface stabilizer. The surface stabilizers preferably are adsorbed on, or associated with, the surface of the posaconazole particles. Surface stabilizers especially useful herein preferably physically adhere on, or associate with, the surface of the nanoparticulate posaconazole particles, but do not chemically react with the posaconazole particles or itself. Individually adsorbed molecules of the surface stabilizer are essentially free of intermolecular cross-linkages.

The present invention also includes posaconazole, or a salt or derivative thereof, compositions together with one or more non-toxic physiologically acceptable carriers, adjuvants, or vehicles, collectively referred to as carriers. The compositions can be formulated for parenteral injection (e.g., intravenous, intramuscular, or subcutaneous), oral administration in solid, liquid, or aerosol form, vaginal, nasal, rectal, ocular, local (powders, ointments or drops), buccal, intracisternal, intraperitoneal, or topical administration, and the like.

### 1. Posaconazole Particles

The compositions of the invention comprise particles of posaconazole or a salt or derivative thereof. The particles can be in a crystalline phase, semi-crystalline phase, amorphous phase, semi-amorphous phase, or a combination thereof.

### 2. Surface Stabilizers

Combinations of more than one surface stabilizers can be used in the invention. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, ionic, anionic, cationic, and zwitterionic surfactants or compounds.

Representative examples of surface stabilizers include albumin (including for example bovine serum albumin and human serum albumin), hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., the commercially available Tweens^{®} such as e.g., Tween 20^{®} and Tween 80^{®} (ICI Speciality Chemicals)); polyethylene glycols (e.g., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (e.g., Pluronics F68^{®} and F108^{®}, which are block copolymers of ethylene oxide and propylene oxide); poloxamines (e.g., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quatemium-15), distearyldimonium chloride (Quatemium-5), dodecyl dimethyl ethylbenzyl ammonium chloride(Quaternium-14), Quatemium-22, Quatemium-26, Quatemium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of para-hydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Posaconazole Particle Size

The compositions of the invention comprise nanoparticulate posaconazole, or a salt or derivative thereof, particles which have an effective average particle size of less than about 2000 nm (*i.e.,* 2 microns), less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the posaconazole particles have a particle size of less than the effective average, by weight (or by other suitable measurement technique, such as by number, volume, etc.), *i.e.,* less than about 2000 nm, 1900 nm, 1800 nm, *etc.,* when measured by the above-noted techniques. In other embodiments of the invention, at least about 60%, at least about 70%, at least about 80%, at least about 90%, at least about 95%, or at least about 99% of the posaconazole particles have a particle size of less than the effective average, *i.e.,* less than about 2000 nm, 1900 nm, 1800 nm, 1700 nm, *etc.*

In the present invention, the value for D50 of a nanoparticulate posaconazole composition is the particle size below which 50% of the posaconazole particles fall, by weight (or by other suitable measurement technique). Similarly, D90 is the particle size below which 90% of the posaconazole particles fall, by weight (or by other suitable measurement technique).

### 5. Concentration of Posaconazole and Surface Stabilizers

The relative amounts of posaconazole, or a salt or derivative thereof, and one or more surface stabilizers can vary widely. The optimal amount of the individual components can depend, for example, upon the particular posaconazole selected, the hydrophilic lipophilic balance (HLB), melting point, and the surface tension of water solutions of the stabilizer, *etc*.

The concentration of the posaconazole can vary from about 99.5% to about 0.001%, from about 95% to about 0.1%, or from about 90% to about 0.5%, by weight, based on the total combined weight of the posaconazole and at least one surface stabilizer, not including other excipients.

The concentration of the at least one surface stabilizer can vary from about 0.5% to about 99.999%, from about 5.0% to about 99.9%, or from about 10% to about 99.5%, by weight, based on the total combined dry weight of the posaconazole and at least one surface stabilizer, not including other excipients.

### 6. Exemplary Nanoparticulate Posaconazole Tablet Formulations

Several exemplary posaconazole tablet formulations are given below. These examples are not intended to limit the claims in any respect, but rather to provide exemplary tablet formulations of posaconazole which can be utilized in the methods of the invention. Such exemplary tablets can also comprise a coating agent.

| **Table 1: Exemplary Nanoparticulate Posaconazole Tablet Formulation #1** | |
|---|---|
| **Component** | **g/Kg** |
| Posaconazole | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

| **Table 2: Exemplary Nanoparticulate Posaconazole Tablet Formulation #2** | |
|---|---|
| **Component** | **g/Kg** |
| Posaconazole | about 100 to about 300 |
| Hypromellose, USP | about 30 to about 50 |
| Docusate Sodium, USP | about 0.5 to about 10 |
| Sucrose, NF | about 100 to about 3 00 |
| Sodium Lauryl Sulfate, NF | about 1 to about 30 |
| Lactose Monohydrate, NF | about 100 to about 3 00 |
| Silicified Microcrystalline Cellulose | about 50 to about 200 |
| Crospovidone, NF | about 50 to about 200 |
| Magnesium Stearate, NF | about 0.5 to about 5 |

| **Table 3: Exemplary Nanoparticulate Posaconazole Tablet Formulation #3** | |
|---|---|
| **Component** | **g/Kg** |
| Posaconazole | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

| **Table 4: Exemplary Nanoparticulate Posaconazole Tablet Formulation #4** | |
|---|---|
| **Component** | **g/Kg** |
| Posaconazole | about 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose | about 129 to about 134 |
| Crospovidone, NF | about 112 to about 118 |
| Magnesium Stearate, NF | about 0.5 to about 3 |

### C. Methods of Making Nanoparticulate Posaconazole Compositions

The nanoparticulate posaconazole, or a salt or derivative thereof, compositions can be made using, for example, milling, homogenization, precipitation, freezing, or template emulsion techniques. Exemplary methods of making nanoparticulate active agent compositions are described in the '684 patent. Methods of making nanoparticulate active agent compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

The resultant nanoparticulate posaconazole compositions or dispersions can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, *etc*.

### 1. Milling to Obtain Nanoparticulate Posaconazole Dispersions

Milling a posaconazole, or a salt or derivative thereof, to obtain a nanoparticulate dispersion comprises dispersing the posaconazole particles in a liquid dispersion medium in which the posaconazole is poorly soluble, followed by applying mechanical means in the presence of grinding media to reduce the particle size of the posaconazole to the desired effective average particle size. The dispersion medium can be, for example, water, safflower oil, ethanol, t-butanol, glycerin, polyethylene glycol (PEG), hexane, or glycol. A preferred dispersion medium is water.

The posaconazole particles can be reduced in size in the presence of at least one surface stabilizer. Alternatively, posaconazole particles can be contacted with one or more surface stabilizers after attrition. Other compounds, such as a diluent, can be added to the posaconazole/surface stabilizer composition during the size reduction process. Dispersions can be manufactured continuously or in a batch mode.

The mechanical means applied to reduce the posaconazole particle size conveniently can take the form of a dispersion mill. Suitable dispersion mills include a ball mill, an attritor mill, a vibratory mill, and media mills such as a sand mill and a bead mill. A media mill is preferred due to the relatively shorter milling time required to provide the desired reduction in particle size. For media milling, the apparent viscosity of the premix is preferably from about 100 to about 1000 centipoise, and for ball milling the apparent viscosity of the premix is preferably from about 1 up to about 100 centipoise. Such ranges tend to afford an optimal balance between efficient particle size reduction and media erosion but are in no way limiting.

Media milling is a high energy milling process. Posaconazole, surface stabilizer, and liquid are placed in a reservoir and recirculated in a chamber containing media and a rotating shaft/impeller. The rotating shaft agitates the media which subjects posaconazole to impaction and sheer forces, thereby reducing the carvedilol particle size.

Ball milling is a low energy milling process that uses milling media, drug, surface stabilizer, and liquid. The materials are placed in a milling vessel that is rotated at optimal speed such that the media cascades and reduces the drug particle size by impaction. The media used must have a high density as the energy for the particle reduction is provided by gravity and the mass of the attrition media.

### Grinding Media

The grinding media for the posaconazole particle size reduction step can be selected from rigid media preferably spherical or particulate in form having an average size less than about 3 mm and, more preferably, less than about 1 mm. Such media desirably can provide the particles of the invention with shorter processing times and impart less wear to the milling equipment. The selection of material for the grinding media is not believed to be critical. Zirconium oxide, such as 95% ZrO stabilized with magnesia, zirconium silicate, ceramic, stainless steel, titania, alumina, 95% ZrO stabilized with yttrium, and glass grinding media are exemplary grinding materials.

The grinding media can comprise particles that are preferably substantially spherical in shape, *e.g*., beads, consisting essentially of polymeric resin or glass or Zirconium Silicate or other suitable compositions. Alternatively, the grinding media can comprise a core having a coating of a polymeric resin adhered thereon.

In general, suitable polymeric resins are chemically and physically inert, substantially free of metals, solvent, and monomers, and of sufficient hardness and friability to enable them to avoid being chipped or crushed during grinding. Suitable polymeric resins include crosslinked polystyrenes, such as polystyrene crosslinked with divinylbenzene; styrene copolymers; polycarbonates; polyacetals, such as Delrin^{®} (E.I. du Pont de Nemours and Co.); vinyl chloride polymers and copolymers; polyurethanes; polyamides; poly(tetrafluoroethylenes), *e.g*., Teflon^{®}(E.I. du Pont de Nemours and Co.), and other fluoropolymers; high density polyethylenes; polypropylenes; cellulose ethers and esters such as cellulose acetate; polyhydroxymethacrylate; polyhydroxyethyl acrylate; and silicone-containing polymers such as polysiloxanes and the like. The polymer can be biodegradable. Exemplary biodegradable polymers include poly(lactides), poly(glycolide) copolymers of lactides and glycolide, polyanhydrides, poly(hydroxyethyl methacylate), poly(imino carbonates), poly(N-acylhydroxyproline)esters, poly(N-palmitoyl hydroxyproline) esters, ethylene-vinyl acetate copolymers, poly(orthoesters), poly(caprolactones), and poly(phosphazenes). For biodegradable polymers, contamination from the media itself advantageously can metabolize *in vivo* into biologically acceptable products that can be eliminated from the body. The polymeric resin can have a density from about 0.8 to about 3.0 g/cm³.

The grinding media preferably ranges in size from about 0.01 to about 3 mm. For fine grinding, the grinding media is preferably from about 0.02 to about 2 mm, and more preferably from about 0.03 to about 1 mm in size.

In one embodiment of the invention, the posaconazole particles are made continuously. Such a method comprises continuously introducing posaconazole into a milling chamber, contacting the posaconazole with grinding media while in the chamber to reduce the posaconazole particle size, and continuously removing the nanoparticulate posaconazole from the milling chamber.

The grinding media can be separated from the milled nanoparticulate posaconazole using conventional separation techniques, in a secondary process such as by simple filtration, sieving through a mesh filter or screen, and the like. Other separation techniques such as centrifugation may also be employed. Alternatively, a screen can be utilized during the milling process to remove the grinding media following completion of particle size reduction.

### 2. Precipitation to Obtain Nanoparticulate Posaconazole Compositions

Another method of forming the desired nanoparticulate posaconazole, or a salt or derivative thereof, composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving the posaconazole in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nanoparticulate Posaconazole Compositions

Exemplary homogenization methods of preparing active agent nanoparticulate compositions are described in U.S. Patent No. 5,510,118, for "Process of Preparing Therapeutic Compositions Containing Nanoparticles." Such a method comprises dispersing particles of a posaconazole, or a salt or derivative thereof, in a liquid dispersion medium, followed by subjecting the dispersion to homogenization to reduce the particle size of a posaconazole to the desired effective average particle size. The posaconazole particles can be reduced in size in the presence of at least one surface stabilizer. Alternatively, the posaconazole particles can be contacted with one or more surface stabilizers either before or after attrition. Other compounds, such as a diluent, can be added to the posaconazole/surface stabilizer composition either before, during, or after the size reduction process. Dispersions can be manufactured continuously or in a batch mode.

### 4. Cryogenic Methodologies to Obtain Nanoparticulate Posaconazole Compositions

Another method of forming the desired nanoparticulate posaconazole, or a salt or derivative thereof, composition is by spray freezing into liquid (SFL). This technology comprises an organic or organoaqueous solution of posaconazole with stabilizers, which is injected into a cryogenic liquid, such as liquid nitrogen. The droplets of the posaconazole solution freeze at a rate sufficient to minimize crystallization and particle growth, thus formulating nanostructured posaconazole particles. Depending on the choice of solvent system and processing conditions, the nanoparticulate posaconazole particles can have varying particle morphology. In the isolation step, the nitrogen and solvent are removed under conditions that avoid agglomeration or ripening of the posaconazole particles.

As a complementary technology to SFL, ultra rapid freezing (URF) may also be used to create equivalent nanostructured posaconazole particles with greatly enhanced surface area.

URF comprises an organic or organoaqueous solution of posaconazole with stabilizers onto a cryogenic substrate.

### 5. Emulsion Methodologies to Obtain Nanoparticulate Posaconazole Compositions

Another method of forming the desired nanoparticulate posaconazole, or a salt or derivative thereof, composition is by template emulsion. Template emulsion creates nanostructured posaconazole particles with controlled particle size distribution and rapid dissolution performance. The method comprises an oil-in-water emulsion that is prepared, then swelled with a non-aqueous solution comprising the posaconazole and stabilizers. The particle size distribution of the posaconazole particles is a direct result of the size of the emulsion droplets prior to loading with the posaconazole a property which can be controlled and optimized in this process. Furthermore, through selected use of solvents and stabilizers, emulsion stability is achieved with no or suppressed Ostwald ripening. Subsequently, the solvent and water are removed, and the stabilized nanostructured posaconazole particles are recovered. Various posaconazole particles morphologies can be achieved by appropriate control of processing conditions.

### 6. Supercritical Fluid Techniques Used to Obtain Nanoparticulate Posaconazole Compositions

Published International Patent Application No. WO 97/14407 to Pace et al., published April 24, 1997, discloses particles of water insoluble biologically active compounds with an average size of 100 nm to 300 nm that are prepared by dissolving the compound in a solution and then spraying the solution into compressed gas, liquid or supercritical fluid in the presence of appropriate surface modifiers. A "supercritical fluid" is any substance at a temperature and pressure above its thermodynamic critical point. Common examples of supercritical fluids include, but are not limited to, carbon dioxide, ethane, ethylene, propane, propylene, trifluoromethane (fluoroform), chlorotrifluoromethane, trichlorofluoromethane, ammonia, water, cyclohexane, n-pentane and toluene.

### 7. Nano-Electrospray Techniques Used to Obtain Nanoparticulate Posaconazole Compositions

In electrospray ionization a liquid is pushed through a very small charged, usually metal, capillary. This liquid contains the desired substance, e.g., posaconazole (or "analyte"), dissolved in a large amount of solvent, which is usually much more volatile than the analyte. Volatile acids, bases or buffers are often added to this solution as well. The analyte exists as an ion in solution either in a protonated form or as an anion. As like charges repel, the liquid pushes itself out of the capillary and forms a mist or an aerosol of small droplets about 10 µm across. This jet of aerosol droplets is at least partially produced by a process involving the formation of a Taylor cone and a jet from the tip of this cone. A neutral carrier gas, such as nitrogen gas, is sometimes used to help nebulize the liquid and to help evaporate the neutral solvent in the small droplets. As the small droplets evaporate, suspended in the air, the charged analyte molecules are forced closer together. The drops become unstable as the similarly charged molecules come closer together and the droplets once again break up. This is referred to as Coulombic fission because it is the repulsive Coulombic forces between charged analyte molecules that drive it. This process repeats itself until the analyte is free of solvent and is a lone ion.

In nanotechnology the electrospray method may be employed to deposit single particles on surfaces, e.g., particles ofposaconazole. This is accomplished by spraying colloids and making sure that on average there is not more than one particle per droplet. Consequent drying of the surrounding solvent results in an aerosol stream of single particles of the desired type. Here the ionizing property of the process is not crucial for the application but may be put to use in electrostatic precipitation of the particles.

### D. Controlled Release Nanoparticulate Posaconazole Formulations

Another aspect of the present invention comprises covering the nanoparticulate posaconazole particles described above in a polymeric coating or matrix. Since the solubility of posaconazole is pH-dependent, the dissolution rate and consequent bioavailability of the drug can change as it passes through different areas of the gastroenterologic system. Coating the particles for a sustained and/or controlled release results in an improved, consistent dissolution rate of the drug which will avoid the occurrence of localized high drug concentrations.

Any coating material which modifies the release of the nanoparticulate posaconazole particles in the desired manner may be used. In particular, coating materials suitable for use in the practice of the invention include but are not limited to polymer coating materials, such as cellulose acetate phthalate, cellulose acetate trimaletate, hydroxy propyl methylcellulose phthalate, polyvinyl acetate phthalate, ammonio methacrylate copolymers such as those sold under the Trade Mark Eudragit® RS and RL, poly acrylic acid and poly acrylate and methacrylate copolymers such as those sold under the Trade Mark Eudragite S and L, polyvinyl acetaldiethylamino acetate, hydroxypropyl methylcellulose acetate succinate, shellac; hydrogels and gel-forming materials, such as carboxyvinyl polymers, sodium alginate, sodium carmellose, calcium carmellose, sodium carboxymethyl starch, poly vinyl alcohol, hydroxyethyl cellulose, methyl cellulose, gelatin, starch, and cellulose based cross-linked polymers--in which the degree of crosslinking is low so as to facilitate adsorption of water and expansion of the polymer matrix, hydoxypropyl cellulose, hydroxypropyl methylcellulose, polyvinylpyrrolidone, crosslinked starch, microcrystalline cellulose, chitin, aminoacryl-methacrylate copolymer (Eudragit® RS-PM, Rohm & Haas), pullulan, collagen, casein, agar, gum arabic, sodium carboxymethyl cellulose, (swellable hydrophilic polymers) poly(hydroxyalkyl methacrylate) (m. wt. about 5 k-5,000 k), polyvinylpyrrolidone (m. wt. about 10 k-360 k), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (m. wt. about 30 k-300 k), polysaccharides such as agar, acacia, karaya, tragacanth, algins and guar, polyacrylamides, Polyox® polyethylene oxides (m. wt. about 100 k-5,000 k), AquaKeep® acrylate polymers, diesters of polyglucan, crosslinked polyvinyl alcohol and poly N-vinyl-2-pyrrolidone, sodium starch glucolate (e.g. Explotab®; Edward Mandell C. Ltd.); hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, polyethylene oxides (e.g. Polyox®, Union Carbide), methyl ethyl cellulose, ethylhydroxy ethylcellulose, cellulose acetate, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers ofmethacrylic acid or methacrylic acid (e.g. Eudragit®, Rohm and Haas), other acrylic acid derivatives, sorbitan esters, natural gums, lecithins, pectin, alginates, ammonia alginate, sodium, calcium, potassium alginates, propylene glycol alginate, agar, and gums such as arabic, karaya, locust bean, tragacanth, carrageens, guar, xanthan, scleroglucan and mixtures and blends thereof. As will be appreciated by the person skilled in the art, excipients such as plasticisers, lubricants, solvents and the like may be added to the coating. Suitable plasticisers include for example acetylated monoglycerides; butyl phthalyl butyl glycolate; dibutyl tartrate; diethyl phthalateacetate trimaletate, hydroxy propyl methylcellulose phthalate, polyvinyl acetate phthalate, dimethyl phthalate; ethyl phthalyl ethyl glycolate; glycerin; propylene glycol; triacetin; citrate; tripropioin; diacetin; dibutyl phthalate; acetyl monoglyceride; polyethylene glycols; castor oil; triethyl citrate; polyhydric alcohols, glycerol, acetate esters, gylcerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitate, diethylhexyl phthalate, di-n-octyl phthalate, di-i-octyl phthalate, di-i-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate and mixtures thereof.

When the modified release component comprises a modified release matrix material, any suitable modified release matrix material or suitable combination of modified release matrix materials may be used. Such materials are known to those skilled in the art. The term "modified release matrix material" as used herein includes hydrophilic polymers, hydrophobic polymers and mixtures thereof which are capable of modifying the release of a posaconazole dispersed therein *in vitro* or *in vivo.* Modified release matrix materials suitable for the practice of the present invention include but are not limited to microcrytalline cellulose, sodium carboxymethylcellulose, hydoxyalkylcelluloses such as hydroxypropylmethylcellulose and hydroxypropylcellulose, polyethylene oxide, alkylcelluloses such as methylcellulose and ethylcellulose, polyethylene glycol, polyvinylpyrrolidone, cellulose acteate, cellulose acetate butyrate, cellulose acteate phthalate, cellulose acteate trimellitate, polyvinylacetate phthalate, polyalkylmethacrylates, polyvinyl acetate and mixture thereof.

### E. Methods of Using the Nanoparticulate Posaconazole Compositions of the Invention

The invention provides a method of increasing bioavailability of a posaconazole, or a salt or derivative thereof, in a subject. Such a method comprises orally administering to a subject an effective amount of a composition comprising a posaconazole. In one embodiment of the invention, the posaconazole composition, in accordance with standard pharmacokinetic practice, has a bioavailability that is about 50% greater than a conventional dosage form, about 40% greater, about 30% greater, about 20% or about 10% greater.

The compositions of the invention are useful in the prevention and treatment of fungal infection and related diseases. Such pathological states include, but are not limited to, include *Candida spp., Cryptococcus neoformans, Aspergillus spp., Rhizopus spp., Blastomyces dermatitidis, Coccidioides immitis, Histoplasma capsulatum, dermatophytes* and *dematiaceous* fungi.

The posaconazole, or a salt or derivative thereof, compounds of the invention can be administered to a subject via any conventional means including, but not limited to, orally, rectally, ocularly, parenterally (*e.g*., intravenous, intramuscular, or subcutaneous), intracisternally, pulmonary, intravaginally, intraperitoneally, locally (*e.g*., powders, ointments or drops), or as a buccal or nasal spray. As used herein, the term "subject" is used to mean an animal, preferably a mammal, including a human or non-human. The terms patient and subject may be used interchangeably.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate posaconazole, or a salt or derivative thereof, compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to a posaconazole, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

One of ordinary skill will appreciate that effective amounts of a posaconazole can be determined empirically and can be employed in pure form or, where such forms exist, in pharmaceutically acceptable salt, ester, or prodrug form. Actual dosage levels of a posaconazole in the nanoparticulate compositions of the invention may be varied to obtain an amount of a posaconazole that is effective to obtain a desired therapeutic response for a particular composition and method of administration. The selected dosage level therefore depends upon the desired therapeutic effect, the route of administration, the potency of the administered posaconazole, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

The following prophetic example is given to illustrate the present invention. It should be understood, however, that the spirit and scope of the invention is not to be limited to the specific conditions or details described in this example but should only be limited by the scope of the claims that follow. All references identified herein, including U.S. patents, are hereby expressly incorporated by reference

### Example 1

The purpose of this example was to prepare a composition comprising a nanoparticulate posaconazole or a salt or derivative thereof.

An aqueous dispersion of 5% (w/w) posaconazole, combined with one or more surface stabilizers, such as hydroxypropyl cellulose (HPC-SL) and dioctylsulfosuccinate (DOSS), could be milled in a 10 ml chamber of a NanoMill® 0.01 (NanoMill Systems, King of Prussia, PA; see e.g., U.S. Patent No. 6,431,478), along with 500 micron PolyMill® attrition media (Dow Chemical Co.) (e.g., at an 89% media load). In an exemplary process, the mixture could be milled at a speed of 2500 rpms for 60 minutes.

Following milling, the particle size of the milled posaconazole particles can be measured, in deionized distilled water, using a Horiba LA 910 particle size analyzer. For a successful composition, the initial mean and/or D50 milled posaconazole particle size is expected to be less than 2000 nm.

It will be apparent to those skilled in the art that various modifications and variations can be made in the methods and compositions of the present inventions without departing from the spirit or scope of the invention. Thus, it is intended that the present invention cover the modification and variations of the invention provided they come within the scope of the appended claims and their equivalents.
The following pages 46 to 53 contain preferred embodiments. Accordingly, the term "claim" as used therein refers to such a "preferred embodiment".
1. A stable nanoparticulate posaconazole composition comprising:
   (a) particles of a posaconazole or a salt or derivative thereof having an effective average particle size of less than about 2000 nm; and
   (b) at least one surface stabilizer.
2. A parenteral dosage form comprising the nanoparticulate posaconazole composition of claim 1.
3. The composition of claim 1, wherein the posaconazole particles are in a crystalline phase, an amorphous phase, a semi-crystalline phase, a semi amorphous phase, or a mixture thereof.
4. The composition of claim 1, wherein the effective average particle size of the posaconazole particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
5. The composition of claim 1, wherein said posaconazole particles have improved bioavailability as compared to non-nanoparticulate posaconazole tablets.
6. The composition of claim 1, wherein the composition is formulated:
   (a) for administration selected from the group consisting of parental injection, oral administration in solid, liquid, or aerosol form, vaginal, nasal, rectal, otically, ocular, local, buccal, intracisternal, intraperitoneal, and topical administration;
   (b) into a dosage form selected from the group consisting of liquid dispersions, gels, sachets, solutions, aerosols, ointments, tablets, capsules, creams, and mixtures thereof;
   (c) into a dosage form selected from the group consisting of controlled release formulations, fast melt formulations, lyophilized formulations, delayed release formulations, extended release formulations, pulsatile release formulations, and mixed immediate release and controlled release formulations; or
   (d) any combination thereof.
7. The composition of claim 1, wherein the composition further comprises one or more pharmaceutically acceptable excipients, carriers, or a combination thereof.
8. The composition of claim 1, wherein:
   (a) posaconazole is present in an amount consisting of from about 99.5% to about 0.001%, from about 95% to about 0.1%, and from about 90% to about 0.5%, by weight, based on the total combined weight of posaconazole and at least one surface stabilizer, not including other excipients;
   (b) at least one surface stabilizer is present in an amount of from about 0.5% to about 99.999% by weight, from about 5.0% to about 99.9% by weight, and from about 10% to about 99.5% by weight, based on the total combined dry weight of posaconazole and at least one surface stabilizer, not including other excipients; or
   (c) a combination thereof.
9. The composition of claim 1, comprising at least two surface stabilizers.
10. The composition of claim 1, wherein the surface stabilizer is selected from the group consisting of an ionic surface stabilizer, anionic surface stabilizer, a cationic surface stabilizer, a zwitterionic surface stabilizer, and a nonionic surface stabilizer.
11. The composition of claim 1, wherein the surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, lysozyme, random copolymers of vinyl acetate and vinyl pyrrolidone, a cationic polymer, a cationic biopolymer, a cationic polysaccharide, a cationic cellulosic, a cationic alginate, a cationic non-polymeric compound, a cationic phospholipid, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL™, ALKAQUAT™, alkyl pyridinium salts; amines, amine salts, amine oxides, imide azolinium salts, protonated quaternary acrylamides, methylated quaternary polymers, and cationic guar.
12. The composition claim 1, wherein:
   (a) upon administration to a mammal the particles of posaconazole or a salt or derivative thereof redisperse such that the particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 mn, less than about 400 nm, less than about 300 nm, less than about 250 nm, less than about 200 nm, less than about 150 mn, less than about 100 nm, less than about 75 nm, and less than about 50 nm;
   (b) the particles of posaconazole or a salt or derivative thereof redisperse in a biorelevant media such that the particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm; or
   (c) a combination of (a) and (b).
13. The composition of claim 12, wherein the biorelevant media is selected from the group consisting of water, aqueous electrolyte solutions, aqueous solutions of a salt, aqueous solutions of an acid, aqueous solutions of a base, and combinations thereof.
14. The composition of claim 1, wherein:
   (a) the Tₘₐₓ of posaconazole or a salt or derivative thereof, when assayed in the plasma of a mammalian subject following administration, is less than the Tₘₐₓ for a non-nanoparticulate composition of the same posaconazole, administered at the same dosage;
   (b) the Cₘₐₓ of posaconazole or a salt or derivative thereof, when assayed in the plasma of a mammalian subject following administration, is greater than the Cₘₐₓ for a non-nanoparticulate composition of the same posaconazole, administered at the same dosage;
   (c) the AUC of posaconazole or a salt or derivative thereof, when assayed in the plasma of a mammalian subject following administration, is greater than the AUC for a non-nanoparticulate composition of the same posaconazole, administered at the same dosage; or
   (d) any combination thereof.
15. The composition of claim 14, wherein:
   (a) the Tₘₐₓ is selected from the group consisting of not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, and not greater than about 5% of the Tₘₐₓ exhibited by a non-nanoparticulate composition of the same posaconazole, administered at the same dosage;
   (b) the Cₘₐₓ is selected from the group consisting of at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by a non-nanoparticulate composition of the same posaconazole, administered at the same dosage;
   (c) the AUC is selected from the group consisting of at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate formulation of the same posaconazole, administered at the same dosage; or
   (d) any combination thereof.
16. The composition of claim 1, wherein the composition does not produce significantly different absorption levels when administered under fed as compared to fasting conditions.
17. The composition of claim 16, wherein the difference in absorption of the posaconazole, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%.
18. The composition of claim 1, wherein the pharmacokinetic profile of the composition is not significantly affected by the fed or fasted state of a subject ingesting the composition.
19. The composition of claim 1, wherein administration of the composition to a human in a fasted state is bioequivalent to administration of the composition to a subject in a fed state.
20. The composition of claim 19, wherein "bioequivalency" is established by:
   (a) a 90% Confidence Interval of between 0.80 and 1.25 for both Cₘₐₓ and AUC; or
   (b) a 90% Confidence Interval of between 0.80 and 1.25 for AUC and a 90% Confidence Interval of between 0.70 to 1.43 for Cₘₐₓ.
21. The composition of claim 1, additionally comprising one or more active agents useful for the prevention and treatment of fungal infection and related diseases.
22. A method for preparing a nanoparticulate posaconazole, or a salt or derivative thereof, composition comprising contacting posaconazole particles with at least one surface stabilizer for a time and under conditions sufficient to provide a nanoparticulate posaconazole composition having an effective average particle size of less than about 2000 nm.
23. The method of claim 22, wherein the contacting comprises grinding, wet grinding, homogenization, freezing, template emulsion, precipitation, or a combination thereof.
24. The method of claim 22, wherein the effective average particle size of the posaconazole particles is selected from the group consisting of less than about 1900 nm, less than about 1800 nm, less than about 1700 nm, less than about 1600 nm, less than about 1500 nm, less than about 1000 nm, less than about 1400 nm, less than about 1300 nm, less than about 1200 nm, less than about 1100 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
25. A method for preventing and/or treating a fungal infection or related disease comprising administering a nanoparticulate posaconazole composition comprising:
   (a) particles of a posaconazole or a salt or derivative thereof having an effective average particle size of less than about 2000 nm; and
   (b) at least one surface stabilizer.
26. The method of claim 25, wherein the effective average particle size of the posaconazole particles is selected from the group consisting of less than about 1900 nm, less than about 1800 nm, less than about 1700 nm, less than about 1600 nm, less than about 1500 nm, less than about 1000 nm, less than about 1400 nm, less than about 1300 nm, less than about 1200 nm, less than about 1100 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

## Claims

1. A stable nanoparticulate posaconazole composition formulated for parenteral administration, comprising:
(a) particles of a posaconazole or a salt or derivative thereof having an effective average particle size of less than 1000 nm; and
(b) at least one surface stabilizer.

2. The composition of claim 1, formulated for intravenous administration.

3. The composition of claim 1 or claim 2, wherein the posaconazole particles are in a crystalline phase, an amorphous phase, a semi-crystalline phase or a mixture thereof.

4. The composition of any of claims 1 to 3, wherein the effective average particle size of the posaconazole particles is selected from the group consisting of less than 900 nm, less than 800 nm, less than 700 nm, less than 600 nm, less than 500 nm, less than 400 nm, less than 300 nm, less than 250 nm, less than 200 nm, less than 100 nm, less than 75 nm, and less than 50 nm.

5. The composition of any of claims 1 to 4, wherein said posaconazole particles have improved bioavailability as compared to non-nanoparticulate posaconazole.

6. The composition of any of claims 1 to 5, wherein the composition further comprises one or more pharmaceutically acceptable excipients, carriers, or a combination thereof.

7. The composition of any of claims 1 to 6, wherein:
(a) posaconazole is present in an amount consisting of from 99.5% to 0.001%, from 95% to 0.1%, and from 90% to 0.5%, by weight, based on the total combined weight of posaconazole and at least one surface stabilizer, not including other excipients;
(b) at least one surface stabilizer is present in an amount of from 0.5% to 99.999% by weight, from 5.0% to 99.9% by weight, and from 10% to 99.5% by weight, based on the total combined dry weight of posaconazole and at least one surface stabilizer, not including other excipients.

8. The composition of any of claims 1 to 7, comprising at least two surface stabilizers.

9. The composition of any of claims 1 to 8, wherein the surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl [beta]-D-glucopyranoside; n-decyl [beta]-D-maltopyranoside; n-dodecyl [beta]-D-glucopyranoside; n-dodecyl [beta]-D-maltoside; heptanoyl-N-methylglucamide; n-heptyl-[beta]-D- glucopyranoside; n-heptyl [beta]-D-thioglucoside; n-hexyl [beta]-D-glucopyranoside; nonanoyl-N-methylglucamide; n-noyl [beta]-D-glucopyranoside; octanoyl-N- methylglucamide; n-octyl-[beta]-D-glucopyranoside; octyl [beta]-D-thioglucopyranoside; lysozyme, PEG-phospholipid, PEG-cholesterol, PEG-cholesterol derivative, PEG- vitamin A, PEG-vitamin E, lysozyme, random copolymers of vinyl acetate and vinyl pyrrolidone, a cationic polymer, a cationic biopolymer, a cationic polysaccharide, a cationic cellulosic, a cationic alginate, a cationic non-polymeric compound, a cationic phospholipid, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, alkyl pyridinium salts; amines, amine salts, amine oxides, imide azolinium salts, protonated quaternary acrylamides, methylated quaternary polymers, and cationic guar.

10. The composition of any of claims 1 to 9, wherein:
(a) the Tₘₐₓ of posaconazole or a salt or derivative thereof, when assayed in the plasma of a mammalian subject following administration is selected from the group consisting of not greater than 90%, not greater than 80%, not greater than 70%, not greater than 60%, not greater than 50%, not greater than 30%, not greater than 25%, not greater than 20%, not greater than 15%, not greater than 10%, and not greater than 5% of the Tₘₐₓ exhibited by a non-nanoparticulate composition of the same posaconazole, administered at the same dosage;
(b) the Cₘₐₓ of posaconazole or a salt or derivative thereof, when assayed in the plasma of a mammalian subject following administration is selected from the group consisting of at least 50%, at least 100%, at least 200%, at least 300%, at least 400%, at least 500%, at least 600%, at least 700%, at least 800%, at least 900%, at least 1000%, at least 1100%, at least 1200%, at least 1300%, at least 1400%, at least 1500%, at least 1600%, at least 1700%, at least 1800%, or at least 1900% greater than the Cₘₐₓ exhibited by a non-nanoparticulate composition of the same posaconazole, administered at the same dosage;
(c) the AUC of posaconazole or a salt or derivative thereof, when assayed in the plasma of a mammalian subject following administration is selected from the group consisting of at least 25%, at least 50%, at least 75%, at least 100%, at least 125%, at least 150%, at least 175%, at least 200%, at least 225%, at least 250%, at least 275%, at least 300%, at least 350%, at least 400%, at least 450%, at least 500%, at least 550%, at least 600%, at least 750%, at least 700%, at least 750%, at least 800%, at least 850%, at least 900%, at least 950%, at least 1000%, at least 1050%, at least 1100%, at least 1150%, or at least 1200% greater than the AUC exhibited by the non-nanoparticulate formulation of the same posaconazole, administered at the same dosage; or
(d) any combination thereof.

11. The composition of any of claims 1 to 10, additionally comprising one or more active agents useful for the prevention and treatment of fungal infection and related diseases.

12. A method for preparing a nanoparticulate posaconazole, or a salt or derivative thereof, parenteral composition comprising contacting posaconazole particles with at least one surface stabilizer for a time and under conditions sufficient to provide a nanoparticulate posaconazole composition having an effective average particle size of less than 1000 nm.

13. The method of claim 12, wherein the contacting comprises grinding, wet grinding, homogenization, freezing, template emulsion, precipitation, or a combination thereof.

14. A nanoparticulate posaconazole composition according to any of claims 1 to 11, for use in preventing and/or treating a fungal infection or related disease.
